Europäisches Patentamt

European Patent Office

Office européen des brevets

(19)

(11) Veröffentlichungsnummer: **0 109 645**

**A2**

(12) # EUROPÄISCHE PATENTANMELDUNG

(21) Anmeldenummer: 83111394.9

(22) Anmeldetag: 15.11.83

(51) Int. Cl.³: **C 07 C 29/15**
**C 07 C 29/34, C 07 C 31/04**
**C 07 C 31/08**

(30) Priorität: 19.11.82 DE 3242697

(43) Veröffentlichungstag der Anmeldung:
30.05.84 Patentblatt 84/22

(84) Benannte Vertragsstaaten:
DE FR GB IT NL

(71) Anmelder: Ruhrchemie Aktiengesellschaft
Bruchstrasse 219
D-4200 Oberhausen 13(DE)

(72) Erfinder: Cornils, Boy, Dr. Dipl.-Chem.
Friedrich-Ebert-Strasse 45
D-4220 Dinslaken(DE)

(72) Erfinder: Lipps, Wolfgang, Dr. Dipl.-Chem.
Falkestrasse 76
D-4200 Oberhausen 11(DE)

(74) Vertreter: Reichelt, Karl-Heinz, Dr.
m. Br. Ruhrchemie Aktiengesellschaft Abt. PLD Postfach
13 01 60
D-4200 Oberhausen 13(DE)

(54) Verfahren zur gleichzeitigen Herstellung von Methanol und Ethanol.

(57) Das bei der Umsetzung von Synthesegas zu Methanol
nach Abtrennung des Methanols verbleibende Restgas wird
mit Methanol katalytisch zu Ethanol umgesetzt.

Croydon Printing Company Ltd.

Ruhrchemie Aktiengesellschaft, Oberhausen 13

## Verfahren zur gleichzeitigen Herstellung von Methanol und Ethanol

Die vorliegende Erfindung betrifft ein Verfahren zur gleichzeitigen Herstellung von Methanol und Ethanol. Hierzu wird durch Vergasung fester und flüssiger Brennstoffe mittels Wasserdampf und Sauerstoff bei höheren Temperaturen und hohen Drücken hergestelltes Synthesegas abgekühlt, teilweise konvertiert und nach Reinigung zu Methanol umgesetzt. Das Methanol wird abgetrennt und mit dem verbleibenden Restgas unter erhöhtem Druck bei erhöhter Temperatur katalytisch zu Ethanol umgesetzt.

Für die Herstellung von Methanol aus Synthesegas sind verschiedene Verfahren bekannt. Das Hochdruckverfahren arbeitet bei einem Synthesegasdruck von etwa 300 bar unter Einsatz von Zinkoxid-Chromoxid-Katalysatoren. In Gegenwart kupferhaltiger Katalysatoren ist die Synthese auch bei niedrigen Drücken, d.h. bei etwa 50 bis 100 bar durchzuführen (vgl. Ullmanss Encyclopädie der Technischen Chemie, 4. Auflage, Bd. 16, Seite 621 ff).

Der wichtigste Rohstoff für die Herstellung von Methanol ist Erdgas, das mit Wasserdampf in einem Steam-Reformer in Synthesegas überführt wird. Das auf diesem Weg erzeugte Synthesegas enthält, bezogen auf die Kohlenoxide, einen Wasserstoff-Überschuß für die Umsetzung zu Methanol. Dieser Wasserstoff-Überschuß bedingt einen relativ hohen

Restgas-Anfall (Purgegas) in der Methanolsynthese. Es muß aus dem Gaskreislauf der Methanolsynthese abgezogen werden und dient im allgemeinen zur Unterfeuerung des Steam-Reformers, was einen Wirkungsgradverlust bedeutet.

Ein anderer Weg zur Herstellung von Synthesegas für die Methanolsynthese geht von Kohle als Rohstoff aus. Je nach angewandtem Verfahren erhält man unterschiedliche Gaszusammensetzungen. Ein für die Methanolsynthese besonders geeignetes Synthesegas wird nach dem Vergasungsverfahren der Texaco (vgl. R. Dürrfeld et al., Chem. Ind. 24 (1982), 312 ff) gewonnen. Es enthält neben einem hohen CO-Überschuß nur 0,1 % Methan. Die Verwendung dieses Gases in der Methanolsynthese führt zu nur geringen Restgasmengen.

Der unmittelbare Einsatz des Restgases als Heizgas ist unwirtschaftlich. Nach einem bekannten Verfahren (DE-OS 24 45 885) wandelt man es daher katalytisch in Methan um, das als hochkaloriges Heizgas verwendet wird. Entsprechend einer anderen Arbeitsweise wird das methanreiche, nach der Methanolabtrennung verbleibende Restgas (Purgegas) unter Druck und bei höherer Temperatur mit Wasserdampf katalytisch zu Wasserstoff und Kohlenoxiden gespalten und dieses Spaltgas in den Kreislauf zur Methanolsynthese zurückgeführt (DE-OS 24 45 884).

Die bekannte Verwendung des Purgegases der Methanolsynthese als Heizgas zur Umwandlung in Methan oder zur Spaltung in Wasserstoff und Kohlenoxid stellt keine optimale Nutzung des in diesem Gas enthaltenen Kohlenmonoxids und Wasserstoffs sicher. In allen Fällen wird dieses Gasgemisch nicht direkt in Wertprodukte umgewandelt, sondern entweder verbrannt oder aber vor der Nutzung einer katalytischen Umsetzung unterworfen.

Der Erfindung liegt die Aufgabe zugrunde, die geschilderten Nachteile zu vermeiden und ein Verfahren bereitzustellen, das es ermöglicht, wirtschaftlich und technisch einfach die Herstellung von Methanol mit der Gewinnung von Ethanol zu verbinden, um so das eingesetzte Synthesegas weitgehend zu nutzen.

Die Erfindung besteht in einem Verfahren zur gleichzeitigen Herstellung von Methanol und Ethanol durch katalytische Umsetzung von Synthesegas zu Methanol und Abtrennung des Methanols aus dem Reaktionsgemisch. Es ist dadurch gekennzeichnet, daß das abgetrennte Methanol zusammen mit dem bei der Abtrennung des Methanols verbleibenden Restgas bei einem Druck von 20 bis 60 MPa und einer Temperatur von 150 bis 250°C zu Ethanol umgesetzt wird.

Der neue Prozeß zeichnet sich dadurch aus, daß der größte Teil des eingesetzten Synthesegases in die Wertprodukte Methanol und Ethanol überführt wird, ohne daß Teile des Synthesegases nur zu Heizzwecken abgegeben werden oder einem aufwendigen Rückgewinnungsverfahren unterworfen werden müssen.

Die Herstellung des Synthesegases kann nach bekannten Verfahren aus kohlenstoffhaltigem Einsatzmaterial, wie Erdöl, Erdgas oder Steinkohle, erfolgen. Besonders geeignet sind Verfahren, die ein methanarmes Synthesegas ergeben. Als Beispiel für ein solches Verfahren sei die Vergasung von Kohle in Form einer wäßrigen Suspension bei Temperaturen von 1200 bis 1600°C und Drücken von 0,5 bis 8 MPa (Texaco-Verfahren) genannt. Anschließend wird das rohe Synthesegas teilweise konvertiert, um das $CO/H_2$-Verhältnis auf den für die Methanol-Synthese geeigneten Wert einzustellen. Nach Reinigung leitet man dann

- 4 -

das Gas dem Methanol-Reaktor zu, in dem die Umsetzung zu Methanol nach dem Hochdruck- oder Niederdruck-Verfahren erfolgt.

Das bei der Methanol-Synthese anfallende Restgas wird anschließend mit Methanol durch ein neues Verfahren, der Homologisierung zu Ethanol, umgesetzt. Zur Einstellung des für die Homologisierungsreaktion erforderlichen Volumverhältnisses von CO und $H_2$, das CO : $H_2$ = 1 : 1 bis 1 : 3 betragen muß, setzt man dem Restgas frisches konvertiertes Synthesegas zu. Falls das Restgas zu wenig Kohlenmonoxid enthält, führt man ihm kohlenmonoxidreiches Synthesegas unmittelbar aus der Vergasung, also vor der Konvertierung, zu.

Die Homologisierung des Methanols mit dem Restgas erfolgt bei Drücken von 20 bis 60 MPa und Temperaturen von 150 bis 250 $^o$C. Als Katalysatoren verwendet man Kobaltkatalysatoren, die durch Halogen in elementarer Form wie Jod und/oder Halogenide wie Chloride, Bromide oder Jodide aktiviert sind und die zusätzlich als Kokatalysatoren Übergangsmetalle der 8. Gruppe des Periodensystems der Elemente, insbesondere Eisen, Ruthenium, Rhodium und Platin sowie als Promotoren organische Verbindungen von Elementen der 5. Hauptgruppe des Periodensystems, vorzugsweise organische Verbindungen des Stickstoffs, Phosphors, Arsens oder Antimons enthalten können. Die Reaktion wird absatzweise oder bevorzugt kontinuierlich durchgeführt.

Das nach der Homologisierungsreaktion durch Abtrennung des Ethanols anfallende Restgas, dessen Kohlenmonoxid- und Wasserstoff-Gehalt im Vergleich zum Restgas der Methanolsynthese erheblich erniedrigt wurde, wird zur vollständigen Nutzung teilweise in den Synthesekreislauf zurückgeführt.

Die Erfindung erlaubt es, durch die erstmalige Schaltung eines heterogen katalysierten mit einem homogen katalysierten System in einem zweistufigen Verfahren aus einem einzigen Rohstoff sowohl Methanol als auch Ethanol herzustellen. Dabei wird der eingesetzte kohlenstoffhaltige Rohstoff, insbesondere Steinkohle, weitgehend genutzt, hohe Methanol- und Ethanol-Ausbeuten werden sichergestellt. Das neue Verfahren zeichnet sich durch Einfachheit aus, es kann in bekannten Anlagen durchgeführt werden.

Die Erfindung wird anhand des folgenden Beispiels näher erläutert.

<u>Beispiel</u>

Durch Vergasung von Kohle werden stündlich 10.000 $Nm^3$ Synthesegas der Zusammensetzung

| | | |
|---|---|---|
| CO | 52 | Vol.-% |
| $H_2$ | 36 | " |
| $CO_2$ | 11 | " |
| $CH_4$ | 0,01 | " |
| $H_2S + COS$ | 0,3 | " |
| $N_2$ | 0,6 | " |

erhalten.

Das Gas wird mit Wasserdampf auf das für die Methanolsynthese erforderliche CO : $H_2$-Verhältnis von 1 : 2,2 konvertiert und zur Entfernung überschüssigen Kohlendioxids und schwefelhaltiger Verbindungen einer Gaswäsche zugeführt. Man erhält stündlich 9172 $Nm^3$ Frischgas der Zusammensetzung

| | | |
|---|---|---|
| CO | 29,8 | Vol.-% |
| $H_2$ | 66,1 | " |
| $CO_2$ | 3,4 | " |
| $CH_4$ | 0,01 | " |
| $N_2$ | 0,65 | " |

85 % dieses Frischgases leitet man der Methanolsynthese zu. Es entstehen 2660 kg Methanol sowie 1897 $Nm^3$ Restgas zu Zusammensetzung

| | | |
|---|---|---|
| CO | 20,2 | Vol.-% |
| $H_2$ | 44,2 | " |
| $CO_2$ | 11 | " |
| $CH_4$ | 22 | " |
| $N_2$ | 2,6 | " |

d.h. es fallen noch 478,75 kg CO sowie 74,83 kg $H_2$ an.

Zur Ausnutzung dieser Bestandteile werden die 1897 $Nm^3$ Restgas mit 15 % des nach der Gaswäsche anfallenden Frischgases vermischt, so daß ein für die Ethanolsynthese geeignetes Synthesegas der Zusammensetzung

| | | |
|---|---|---|
| CO | 24,1 | Vol.-% |
| $H_2$ | 53,2 | " |
| $CO_2$ | 7,7 | " |
| $CH_4$ | 12,7 | " |
| $N_2$ | 2,3 | " |

entsteht, das also 992 kg CO und 156 kg $H_2$ enthält.

Dieses Einsatzgas wird mit 998 kg des bei der Methanolsynthese anfallenden Rohmethanols sowie einem Katalysator aus Kobalt, einem Edelmetall als Kokatalysator und einem Halogenaktivator bei 200°C und 550 bar zu 746 kg Ethanol umgesetzt. Bezogen auf eingesetztes Methanol beträgt die Ethanol-Ausbeute 52 %; ferner fallen 6 % n-Propanol, 9 % Acetaldehyd und Acetale, 5 % Ether sowie 6 % Acetate an.

Das Restgas enthält noch 119 kg CO und 31,3 kg $H_2$. Bezogen auf das Restgas aus der Methanolsynthese werden somit 46 % des darin enthaltenen Synthesegases in Ethanol, 5 % in n-Propanol, 8 % in Acetaldehyd und Acetale, 4,4 % in Ether sowie 5,6 % in Acetate überführt.

0109645

Oberhausen 13, 18.11.82
PLD rcht-sei    - R 1943 -

Ruhrchemie Aktiengesellschaft, Oberhausen 13

Patentansprüche

1.) Verfahren zur gleichzeitigen Herstellung von Methanol
und Ethanol durch katalytische Umsetzung von Synthesegas
zu Methanol und Abtrennung des Methanols, dadurch gekennzeichnet, daß das abgetrennte Methanol zusammen mit dem
bei der Abtrennung des Methanols verbleibenden Restgas
und gegebenenfalls nach Zumischung von weiterem frischem
Synthesegas bei einem Druck von 20 bis 60 MPa und einer
Temperatur von 150 bis 250°C in Gegenwart von Katalysatoren zu Ethanol umgesetzt wird.

2.) Verfahren nach Anspruch 1, dadurch gekennzeichnet,
daß das Synthesegas durch Vergasung von Kohle in wässriger Suspension bei Temperaturen von 1200 bis 1600°C und
Drücken von 0,5 bis 8 MPa gewonnen wird.

3.) Verfahren nach Anspruch 1 und 2, dadurch gekennzeichnet,
daß die Umsetzung des Methanols zu Ethanol mit einem
Kohlenmonoxid-Wasserstoff-Gemisch erfolgt, das CO und $H_2$
im Verhältnis 1 : 1 bis 1 : 3 enthält.

4.) Verfahren nach Anspruch 1 bis 3, dadurch gekennzeichnet,
daß die Umsetzung von Methanol zu Ethanol in Gegenwart von
Kobaltkatalysatoren erfolgt, die durch Halogen und/oder
Halogenide aktiviert sind und die zusätzlich als Kokatalysatoren Übergangsmetalle der 8. Gruppe des Periodensystems der Elemente sowie als Promotoren organische
Verbindungen von Elementen der 5. Gruppe des Periodensystems enthalten.

- 2 -

5.) Verfahren nach Anspruch 4, dadurch gekennzeichnet, daß die Kobaltkatalysatoren durch Jod und/oder Chloride, Bromide oder Jodide aktiviert sind.[

6.) Verfahren nach Anspruch 4, dadurch gekennzeichnet, daß die Übergangsmetalle der 8. Gruppe Ruthenium, Rhodium, Platin oder Eisen sind.

7.) Verfahren nach Anspruch 4, dadurch gekennzeichnet, daß die organischen Verbindungen von Elementen der 5. Hauptgruppe organische Verbindungen des Stickstoffs, Phosphors, Arsens oder Antimons sind.